# EUROPEAN PATENT SPECIFICATION

(11) **EP 1 189 565 B1**
(45) Date of publication and mention of the grant of the patent: **23.03.2005**
(21) Application number: 00948522.8
(22) Date of filing: 23.06.2000
(51) Int. Cl.: A61F 13/15

(54) **PROCESS FOR MANUFACTURING DISPOSABLE ABSORBENT CORES, AND AN APPARATUS FOR PERFORMING THE PROCESS**
VERFAHREN ZUM HERSTELLEN VON ABSORBIERENDEN WEGWERFKERNEN, UND VORRICHTUNG ZUR DURCHFÜHRUNG DES VERFAHRENS
PROCEDE DE FABRICATION D'AMES ABSORBANTES JETABLES ET APPAREIL A CET EFFET

(30) Priority: 25.06.1999 EP 99112229
(43) Date of publication of application: 27.03.2002
(73) Proprietor: THE PROCTER & GAMBLE COMPANY, Cincinnati, Ohio 45202 (US)
(72) Inventor: FRANZMANN, Dirk, D-50676 Koln (DE); SCHMITZ, Christoph, Johann, D-53881 Euskirchen-Stotzheim (DE)
(74) Representative: Borbach, Markus
(86) International application number: PCT/US2000/017500
(87) International publication number: WO 2001/000123

(56) References cited:
- EP-A- 0 652 175
- WO-A-96/21411
- US-A- 4 673 402
- US-A- 4 760 764
- US-A- 5 147 345
- US-A- 5 584 954
- US-A- 5 660 665

## Description

The invention relates to a process for manufacturing profiled disposable absorbent cores, without waste of the absorbent core material; and to an apparatus for performing the process. In particular, diapers or training pants may be made by the process of the invention having absorbent cores with an hourglass-shape for better fit around the legs of the wearer, but the process may also be applied to feminine hygiene articles, adult incontinence articles, and other disposable absorbent articles.

Disposable absorbent articles have become very popular in the market place today. Many of these articles include features such as absorbent cores providing a variety of functions including improved containment characteristics and better, more comfortable fit.

An overriding consideration in the construction of a disposable absorbent article is the cost of manufacturing the article, including the materials cost. The present invention provides methods for manufacturing absorbent cores for absorbent articles with little or no wasted material. Thus, the absorbent cores made by the process of the present invention can be provided at relatively lower cost than many of the absorbent cores that are currently manufactured using techniques in which material is wasted. Processes which reduce or avoid material waste are disclosed in the following references.

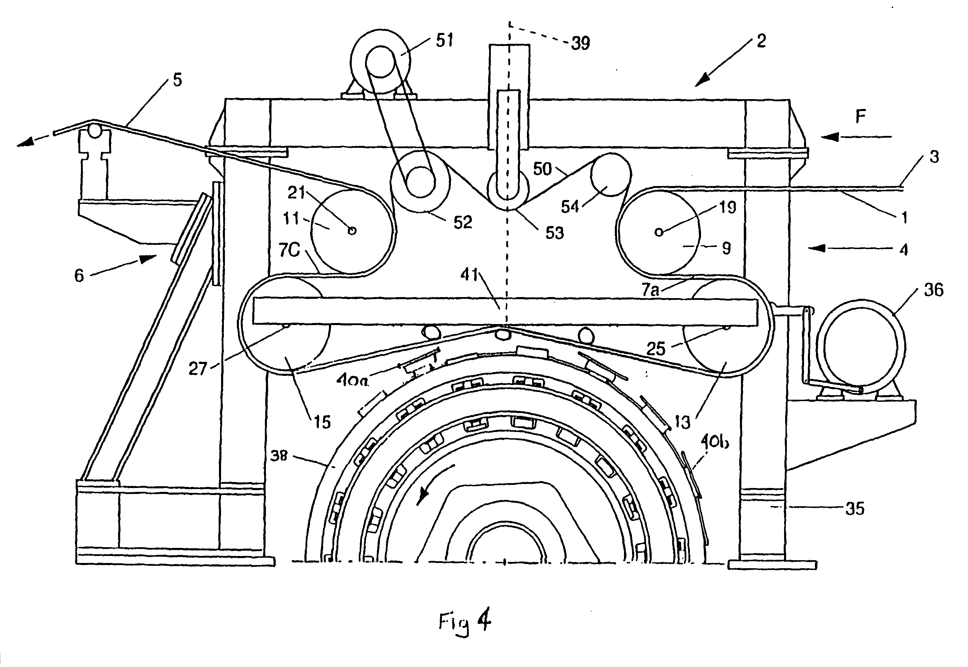

US-A-4 760 764, issued on 2^{nd} August 1988, relates to manufacturing methods for disposable diapers which have an "egg-timer" profile. It is proposed to reduce production costs by making effective use of the absorbent core, and in particular by avoiding waste by cutting out and throwing away a part of the absorbent core. This is achieved by providing "nested panels" in a single side panel web and then rearranging the panels, for example as shown in Figure 4 of the patent to form an absorbent pad.

WO96/21411, published on 18^{th} July 1996, discloses a method for manufacturing shaped or contoured absorbent cores made from multiple pieces or layers. Wherein the multiple pieces or layers are cut from the same continuous web with "zero scrap".

U.S. Patent 5,660,665 discloses an apparatus including a rotating base roll for applying at least one stretched strip of a first material to a base web comprising a second material and moving continuously in first direction. The discrete length strip is cut on the rotating roll from a continuous ribbon, is rotated on the base roll to a desired orientation and is stretched to a desired length. The apparatus includes the rotating roll feed apparatus for feeding the continuous ribbon to a circumferential engagement with the rotating roll, a cutter for cutting the discrete length strips from the continuous ribbon, at least one rotating extensible platen mounted on and rotating with the rotating roll and an applicator rotating in concert with the rotating base roll, for applying the cut, rotated and stretched strips to the base web.

It is an object of the present invention to provide an alternative method of achieving waste-saving benefits. and associated reduction of material costs.

The invention provides a process and apparatus for the manufacture of disposable absorbent cores from a continuous web, wherein a plurality of first and second core elements, are cut from the same continuous web.

### Summary of the Invention

The object of the invention is achieved by rotating each of first and second core elements about axes perpendicular to the plane of the core element and subsequently combining a first core element and a second core element in proximal relationship to form the absorbent core. Preferably each of the first and second core elements are rotated through 90° in mutually counter-rotating directions.

### Brief Description of the Drawings

Figure 1 shows a perspective view of an apparatus for use in the process of the present invention;
Figure 2 shows a side elevation view of the apparatus of Figure 1;
Figures 3a and 3b show rear and front elevation views of the apparatus of Figure 1;
Figure 4 shows a schematic side elevation view of a production line assembly comprising an apparatus of the present invention;
Figure 5a shows a plan view of core elements after cutting, according to one embodiment of the process of the present invention. Figure 5b shows a plan view of an absorbent core made by combining a first core element and a second core element of Figure 5a.

### Detailed Description of the Invention

Incontinence management articles, such as non-cloth disposable diapers, have traditionally utilized absorbent structures which comprise entangled masses of fibers, i.e. non woven fibrous webs. These webs imbibe aqueous fluids, including discharged body fluids, both by an absorption mechanism where fluid is taken up by the fiber material itself, and especially by a wicking mechanism where fluid is acquired by, distributed through and stored in the capillary interstices between the fibers. These webs often comprise loosely compacted, low density layers of absorbent fibers, such as carded cotton webs, air-laid cellulose fibers, comminuted wood pulp fibers, and the like. Fibrous webs used in such absorbent articles also often include certain absorbent gelling materials usually referred to as "hydrogels", "super absorbent" or "hydrocolloid" materials to store large quantities of the discharged body fluids. Known technology to manufacture such cores includes air laying the fibers into shaped cavities on a screened drum to form the shape of the core and control the quantity of material used per core. Excess overfill of the shaped cavities is removed and returned to the incoming air stream by a scarfing roll. The Absorbent Gelling Materials (AGM) are added to the airstream along with the fiber materials. This process allows for many alternative shapes to be produced via changes in screen, laydown drum, configuration. Therefore the shape of the material is achieved via "molding" the fibers into shaped cavities. This process provides shape without trim, which in turn results in minimal scrap. Examples of such cores are disclosed in US-A-4 673 402, issued 16^{th} June 1987 and US-A-4 935 022, issued 19^{th} June 1990.

An alternative absorbent material potentially capable of providing capillary fluid transport would be open-celled polymeric foams. If made appropriately, open-celled polymeric foams could provide features of capillary fluid acquisition, transport and storage required for use in high performance absorbent cores for absorbent articles such as diapers. Absorbent articles containing such foams could possess desirable wet integrity, could provide suitable fit throughout the entire period the article is worn, and could avoid changes in shape during use. In addition, absorbent articles containing such foam structures could be easier to manufacture on a commercial scale. For example, absorbent diaper cores could simply be stamped out of continuous foam sheets and could be designed to have considerably greater integrity and uniformity than air-laid fibrous absorbent cores containing particulate absorbent gelling materials.

Shaped or contoured absorbent cores made from open-celled foam materials are disclosed in US-A-5 147 345, issued 15^{th} September 1992. This patent discloses absorbent cores comprising a fluid acquisition/distribution component that can be fibrous or foam based, as well as fluid storage/redistribution component that comprises a hydrophilic, flexible, open-celled polymeric foam. Figure 9 discloses one such shaped or contoured core having an hourglass-shaped fluid redistribution/storage layer comprising an open-cell absorbent foam. Forming shaped or contoured absorbent cores or layers from foam materials is not without problems. The hourglass-shaped foam layer is typically made from a single rectangular piece of foam. This rectangular piece of foam can be notched, cut or otherwise severed from the hourglass-shaped piece. In carrying out these operations, a significant amount of unusable foam scrap can be created. Indeed it has been found that, in forming hourglass-shaped foam pieces, as much as 15% to 25% of the total foam material used can end up as unusable scrap.

The present invention relates to a process and apparatus for manufacturing an absorbent core having "zero scrap", thus eliminating the steps for disposing or recycling surplus material.

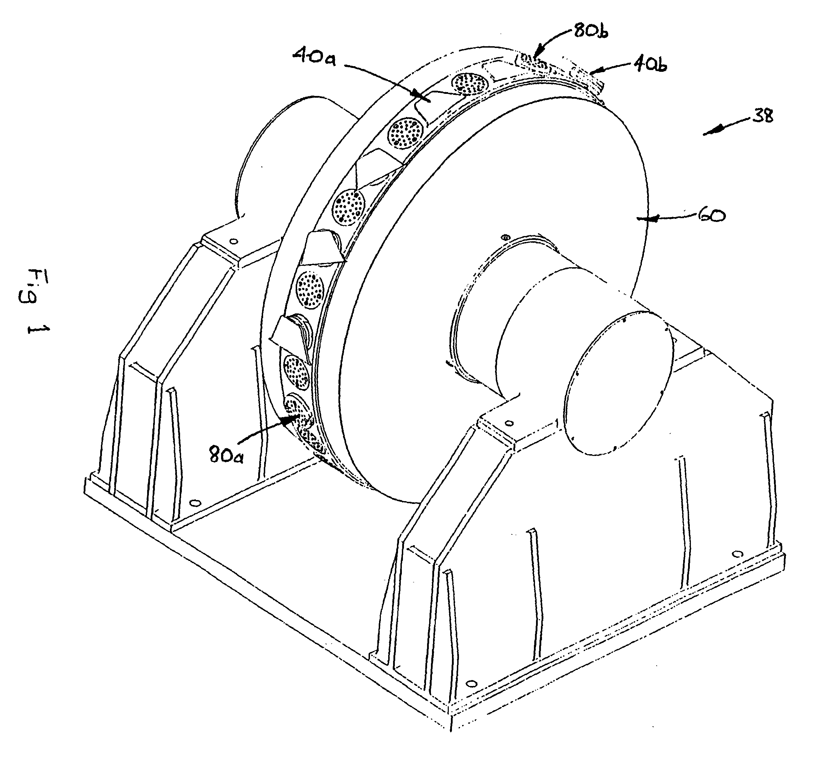

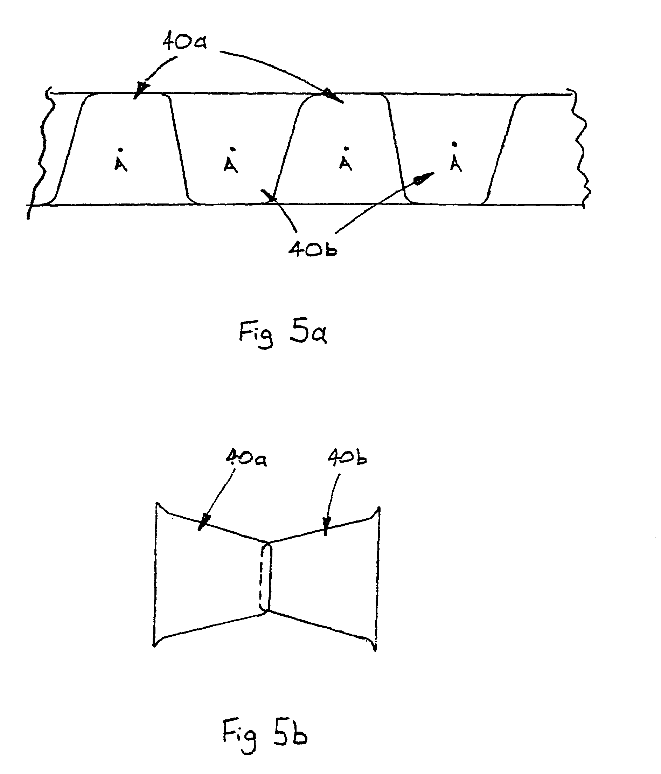

Figure 1 shows a perspective view of an apparatus 38 according to the present invention. The apparatus comprises a rotating drum 60. The drum 60 applies discrete first and second core elements 40a, 40b to a receiving web 1. In Figure 1 an incoming web 40 is fed to the apparatus 38 and is cut in a nested pattern (see, for example, Figure 5), and the discrete first and second core elements 40a, 40b of the incoming webs are held on vacuum shells 80a. 80b which are arranged around the circumference of the rotating drum 60. The receiving web 1; the incoming web 40; and the cutting roll are not shown in Figure 1 to simplify this illustration.

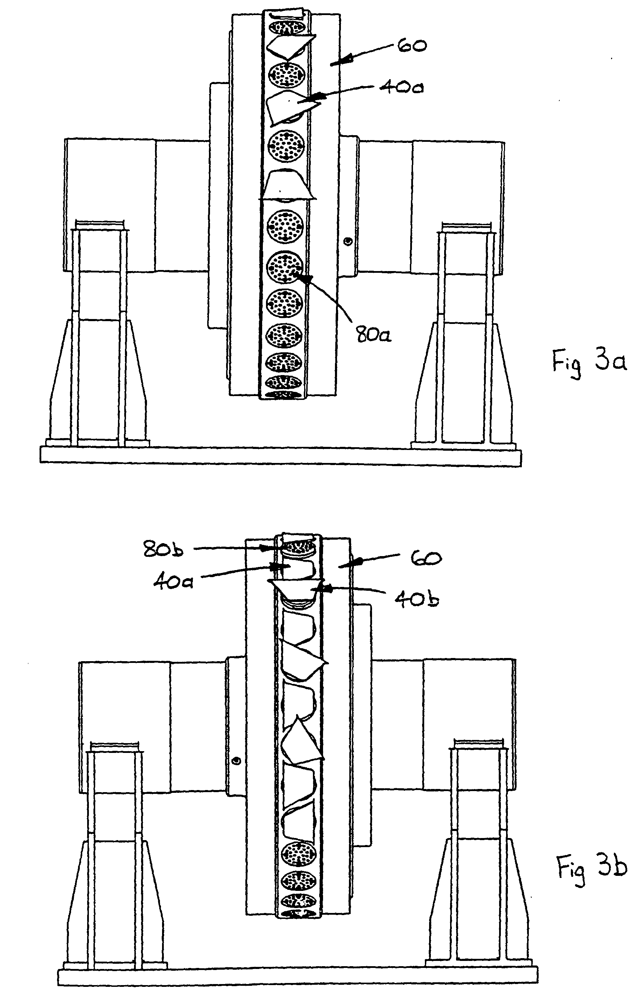

The drum 60 is rotated about a main axis of the apparatus. In the embodiment illustrated in Figures 1, 2, 3a and 3b the main axis is oriented horizontally, but this main axis need not necessarily be horizontal in all cases.

The vacuum shells 80a, 80b are mounted on rotatable shafts 81 and are rotatable about an axis A which is radial with respect to the drum 60. Alternate vacuum shells 80a, 80b are rotatable in mutually counter-rotating directions, preferably plus and minus 90° from the starting position. The means for providing the rotation each of the rotatable shafts 81 may suitably be provided by a rack and pinion arrangement, and a cam follower which runs around a shell turning cam. The shell turning cam is shaped so that the rack and pinion operate to rotate alternate vacuum shells 80b through 90° during a first part of the cycle, prior to transfer of the second core element, 40b of the incoming web to the receiving web 1, and then return the vacuum shells 80b to their original orientation by rotating them back through minus 90° during a second part of the cycle after the transfer.

In addition, during the first part of the cycle, a shell lifting cam acts to "lift" the vacuum shell 80b radially outwards from the drum 60. This action helps to apply the second core element 40b of the incoming web 40 to the receiving web 1 at a transfer step. During the second part of the cycle, after the cut core element 40b of the incoming web has been transferred to the receiving web 1, the vacuum shells 80b are "withdrawn" radially inwards with respect to the drum 60. During the second part of the cycle, or subsequent to the second part of the cycle, the vacuum shells 80a (i.e. those vacuum shells which have not been rotated during the first part of the cycle) are rotated through minus 90° and are "lifted" radially outwards from the drum 60. This action helps to apply the first core elements 40a of the incoming web 40 to the receiving web 1 at a transfer step. Finally the vacuum shells 80a are "withdrawn" radially inwards with respect to the drum 60, and the cycle is ready to repeat.

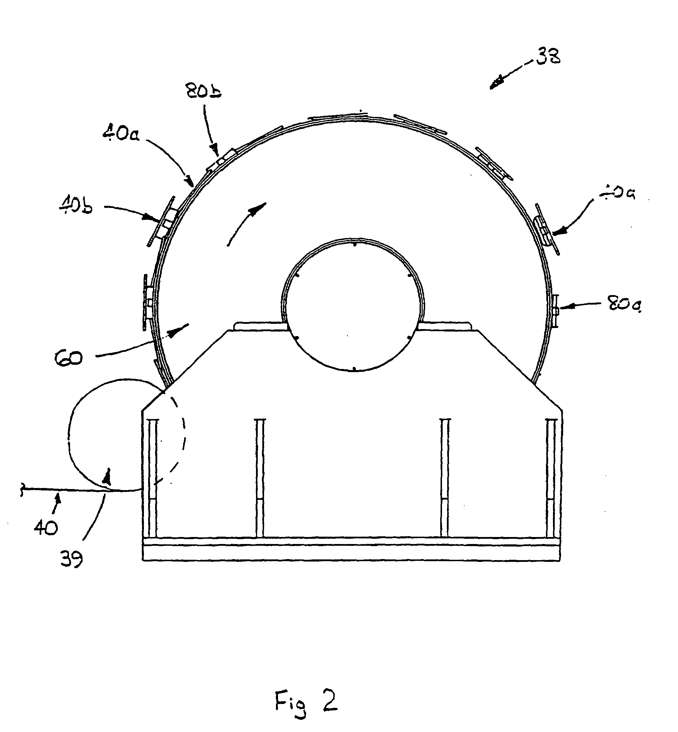

In the embodiment of the invention shown in Figures 1 and 2 the first and second core elements 40a and 40b are rotated through plus 90° and minus 90° respectively in order to provide an hourglass-shaped core suitable for use in an absorbent article such as a diaper.

Typically, an absorbent article is assembled from an absorbent pad element, or core which is encased between a liquid-pervious topsheet and a liquid impervious backsheet. In a preferred embodiment, the cores are comprised of airfelt or foam, within a cellulosic tissue envelope, to provide integrity to the core in use. The backsheet is coated on its inner surface with beads or spirals of adhesive, for affixing the backsheet to the core. Continuous bands of elastic are fed from metering rolls past a glue nozzle. An S-wrap arrangement of the rolls feeding the bands of elastic minimises deformation of the elastic band and allows for accurate control of the speed of the elastic. The elastic bands are fed into the direction of transport at a lower speed than the cores, the backsheet and the topsheet, so that the elastic bands are stretched.

In a particularly preferred embodiment of the present invention a receiving web (which may be, for example, either the backsheet or the topsheet) passes at a constant speed of transport to the infeed side 4 of an assembly 2 for periodically changing the speed of web (illustrated in Figure 4). In the assembly 2, the receiving web 1 can be slowed down, or stopped and is contacted by the apparatus of the invention 38. The apparatus 38 comprises means for providing the core elements. The web 1 leaves the outfeed side 6 of the assembly 2 at the constant web speed. The speed of the receiving web portions located upstream and downstream from the assembly 2 along upstream trajectory 3 and downstream trajectory 5 is not affected by the change in speed of those parts of the receiving web 1 that are passing through the assembly 2.

Figure 4 shows the assembly 2 for changing the speed of a flexible receiving web 1 of relatively low tear strength. By flexible, it is meant that the receiving web 1 can be transported along a curvilinear trajectory and will adapt its shape so as to conform to the trajectory. The receiving web 1 is formed of flexible material, such as paper, airfelt, plastic etc. and can be comprised of the topsheet 121, the backsheet 123 or any combination thereof.

The receiving web 1 is transported along the upstream trajectory 3 with a constant velocity of transport V₀, in the machine direction F. The upstream trajectory 3 is formed by the length of the receiving web 1 which extends to the right of the first guide roller 9 in Figure 4, and which is moving towards the infeed side 4 of the assembly. After passing through the assembly, the receiving web 1 exits at the outfeed side 6 and is transported at constant velocity V₀ along the downstream trajectory 5, which extends to the left of the guide roller 11. The upstream and downstream trajectories need not correspond to the machine direction, and can be formed by straight-line or curvilinear paths.

The guide rollers 9 and 11 are rotationally connected to the frame 35. The guide rollers 9, 11 have a fixed position. The receiving web 1 is looped around an upstream and a downstream transport roller 13, 15 which are mounted on a sled 41. The sled 41 is cyclically translated along the frame 35, generally parallel to the machine direction F, by drive motor 36.

An intermediate trajectory 7a, 7b, 7c of the receiving web 1 is located between the upstream guide roller 9 and the downstream guide roller 11, and comprises a first section 7a and a third section 7c, of variable length, located between the upstream guide roller 9 and the upstream transport roller 13 and the downstream transport roller 15 and the downstream guide roller 11 respectively. The second section 7c of the intermediate trajectory 7 is located between the transport rollers 13 and 15 and is of constant length.

Because of the symmetry of the intermediate trajectory 7a, 7b, 7c, the increase in length of the first section 7a, upon displacement of the sled 41 opposite to the machine direction F and away from the equilibrium position 39, is compensated by an equal decrease in length of the third section 7c, and vice versa. As the length of the second section 7b is constant, the whole intermediate trajectory 7a, 7b, 7c is independent of the position of the sled 41 with respect to the frame 35.

When the part of the receiving web that is located along the second section 7b of the intermediate trajectory 7a, 7b, 7c, is stationary (or at least it is slower than the speed of the web speed V₀) relative to the frame 35, the web 1 is contacted by the applicator means 38 which is positionally stationary (or at least slower) with respect to the frame 35. After the apparatus 38 has interacted with the receiving web 1, the web is accelerated along the section 7b of the intermediate trajectory towards the outfeed side 6 of the assembly 2, and is supplied to the downstream trajectory 5 with web speed V₀.

The guide rollers 9, 11 and the transport rollers 13, 15 are driven by a drive member in the form of a closed loop 50 and pulleys 52, 53 and 54. The loop 50 is partly parallel to the intermediate trajectory 7a, 7b, 7c. The loop 50 is driven at a constant speed which is equal to the speed of transport V₀, of the web 1 by a single drive motor 51. By driving the guide rollers 9, 11 and the transport rollers 13, 15, the strain exerted on the web 1 is minimised and can be limited to the acceleration forces, which are acting to change the speed of the web. Further details of suitable assembly are disclosed in EP-A-0 652 175, published on 10^{th} May 1995.

Figure 5 shows a web 40 which has been cut into discrete core elements 40a, 40b. Second core elements 40b are subsequently rotated about their axis A.

The first and second core elements 40a, 40b are either mutually adhered, or are adhered to the receiving web 1, or both. This may be done using any, conventional method such as by gluing with adhesives, such as melt adhesives, or use of self-adhesive components, or by use of ultrasonic welding, heat sealing or the like.

The transport rollers (13, 15) are periodically displaced relative to the guide rollers (9, 11) around a transfer position preferably at a frequency of between 1Hz and 100 Hz, more preferably between 1Hz and 10 Hz.

## Claims

1. A process for the manufacture of disposable absorbent cores from a continuous web (40), wherein a plurality of first and second core elements (40a, 40b), are cut from the same continuous web (40), and said first and second core elements (40a, 40b) are held on vacuum shells (80a, 80b), said vacuum shells (80a, 80b) being arranged around the circumference of a rotating drum (60), said drum being rotated about a main axis, wherein the absorbent core is formed by rotating each of first and second core elements (40a, 40b) about axes (A) perpendicular to the plane of the core element (40a, 40b) and subsequently combining a first core element (40a) and a second core element (40b) in proximal relationship to form the absorbent core, **characterised in that** a shell lifting cam acts to lift the vacuum shell (80a, 80b) radially outwards from said drum (60) to apply the core elements (40a, 40b) to a receiving web (1) at a transfer step.

2. A process according to claim 1 wherein each of the first and second core elements (40a, 40b) are rotated through 90° in mutually counter-rotating directions.

3. A process according to either of claims 1 or 2 wherein the continuous web (40) is made from air-laid fibers or foam, most preferably foam.

4. A process according to any of claims 1 to 3 wherein a receiving web (1) is fed relative to a stationary frame (35) along an upstream trajectory (3), a downstream trajectory (5), and an intermediate trajectory (7a, 7b, 7c) comprised between the upstream trajectory (3) and the downstream trajectory (5), the web (1) having along the upstream trajectory (3) and along the downstream trajectory (5) a substantially constant speed of transport, wherein the receiving web (1) runs along an upstream and a downstream guide roller (9, 11) that are translationally stationary relative to the frame (35) and along an upstream and downstream transport roller (13, 15) that are periodically displaced relative to the guide rollers (9, 11) around a transfer position (39), and wherein the discrete core elements (40a, 40b) are applied to the receiving web (1) when it is at the transfer position.

5. A process according to claim 4 wherein the periodic displacement of the transport rollers (13, 15) around the transfer position occurs at a frequency of between 1 Hz and 100 Hz, preferably between 1 Hz and 10 Hz.

6. A process according to any of the previous claims wherein the receiving web (1) comprises either a liquid-pervious topsheet or a liquid impervious backsheet.

7. An apparatus (38) for the manufacture of disposable absorbent cores from a continuous web (40), wherein the apparatus comprises a means for cutting a plurality of first and second core elements (40a, 40b) from the same continuous web (40), and the apparatus further comprises vacuum shells (80a, 80b) which are arranged around the circumference of a rotating drum (60), wherein the first and second core elements (40a, 40b) are held on said vacuum shells (80a, 80b), each of the vacuum shells (80b) being rotatable about an axis (A) perpendicular to the plane of the core elements (40a, 40b), the apparatus further comprising a rotating drum (60) to apply discrete first and second core elements (40a, 40b) to a receiving web (1), thereby combining a first core element (40a) and a second core element (40b) in proximal relationship to form the absorbent core, **characterized in that** the apparatus further comprises a shell lifting cam able to lift the vacuum shell (80a, 80b) radially outwards from said drum (60) the apply the core elements (40a, 40b) to the receiving web (1) at a transfer step.

## Patentansprüche

1. Verfahren zur Herstellung wegwerfbarer, absorbierender Kerne aus einer kontinuierlichen Bahn (40), wobei eine Vielzahl erster und zweiter Kern-Elemente (40a, 40b) aus der gleichen kontinuierlichen Bahn (40) geschnitten werden, und wobei die ersten und zweiten Kern-Elemente (40a, 40b) an Vakuum-Mantelflächen (80a, 80b) gehalten werden, wobei die Vakuum-Mantelflächen (80a, 80b) um den Umfang einer Dreh-Walze (60) angeordnet sind, wobei die Walze um eine Hauptachse zur Rotation gebracht wird, wobei der absorbierende Kern gebildet wird durch Drehen jedes ersten und zweiten Kern-Elements (40a, 40b) um zu der Ebene des Kern-Elements (40a, 40b) senkrechten Achsen (A) und nachfolgendes Verbinden eines ersten Kern-Elements (40a) und eines zweiten Kern-Elements (40b) in proximaler Beziehung, um den absorbierenden Kern zu bilden, **dadurch gekennzeichnet, dass** ein Mantelflächen-Hub-Nocken wirkt, um die Vakuum-Mantelfläche (80a, 80b) radial nach außen von der Walze (60) zu heben, um die Kern-Elemente (40a, 40b) bei einem Überführungs-Schritt auf eine Empfangs-Bahn (1) zu bringen.

2. Verfahren nach Anspruch 1, wobei jedes der ersten und zweiten Kern-Elemente (40a, 40b) über 90° in wechselseitig gegenläufigen Richtungen gedreht wird.

3. Verfahren nach Anspruch 1 oder 2, wobei die kontinuierliche Bahn (40) aus Luft-gelegten Fasern oder Schaum, am bevorzugtesten aus Schaum, hergestellt ist.

4. Verfahren nach einem der Ansprüche 1 bis 3, wobei eine Empfangs-Bahn (1) relativ zu einem stationären Gestell (35) entlang einer stromaufwärtigen Trajektorie (3), einer stromabwärtigen Trajektorie (5) und einer dazwischenliegenden Trajektorie (7a, 7b, 7c) geführt wird, die zwischen der stromaufwärtigen Trajektorie (3) und der stromabwärtigen Trajektorie (5) liegt, wobei die Bahn (1) entlang der stromaufwärtigen Trajektorie (3) und entlang der stromabwärtigen Trajektorie (5) eine im Wesentlichen konstante Transport-Geschwindigkeit aufweist, wobei die Empfangs-Bahn (1) läuft entlang einer stromaufwärtigen und einer stromabwärtigen Führungs-Walze (9, 11), die translatorisch stationär relativ zu dem Gestell (35) sind, und entlang einer stromaufwärtigen und einer stromabwärtigen Transport-Walze (13, 15), die periodisch relativ zu den Führungs-Walzen (9, 11) um eine Überführungs-Position (39) versetzt sind, und wobei die diskreten Kern-Elemente (40a, 40b) auf die Empfangs-Bahn (1) gebracht werden, wenn sich diese bei der Überführungs-Position befindet.

5. Verfahren nach Anspruch 4, wobei der periodische Versatz der Transport-Walzen (13, 15) um die Überführungs-Position mit einer Frequenz zwischen 1 Hz und 100 Hz, vorzugsweise zwischen 1 Hz und 10 Hz, stattfindet.

6. Verfahren nach einem der vorherigen Ansprüche, wobei die Empfangs-Bahn (1) entweder eine Flüssigkeits-durchlässige Decklage oder eine Flüssigkeits-undurchlässige Außenlage umfasst.

7. Vorrichtung (38) zur Herstellung absorbierender Wegwerf-Keme aus einer kontinuierlichen Bahn (40), wobei die Vorrichtung ein Mittel zum Schneiden einer Vielzahl erster und zweiter Kern-Elemente (40a, 40b) aus der gleichen kontinuierlichen Bahn (40) umfasst, und wobei die Vorrichtung ferner Vakuum-Mantelflächen (80a, 80b) aufweist, die um den Umfang einer Dreh-Walze (60) angeordnet sind, wobei die ersten und zweiten Kern-Elemente (40a, 40b) an den Vakuum-Mantelflächen (80a, 80b) gehalten werden, wobei jede der Vakuum-Mantelflächen (80b) um eine zu der Ebene der Kern-Elemente (40a, 40b) senkrechten Achse (A) drehbar ist, wobei die Vorrichtung ferner eine Dreh-Walze (60) umfasst, um diskrete erste und zweite Kein-Elemente (40a, 40b) auf eine Empfangs-Bahn (1) zu bringen, wodurch ein erstes Kern-Element (40a) und ein zweites Kern-Element (40b) in proximaler Beziehung verbunden werden, um den absorbierenden Kern zu bilden, **dadurch gekennzeichnet, dass** die Vorrichtung ferner einen Mantelflächen-Hub-Nocken umfasst, der imstande ist, die Vakuum-Mantelfläche (80a, 80b) radial nach außen von der Walze (60) zu heben, um die Kern-Elemente (40a, 40b) auf die Empfangs-Bahn (1) bei einem Überführungs-Schritt zu bringen.

## Revendications

1. Procédé pour la fabrication d'âmes absorbantes jetables à partir d'une nappe continue (40), dans lequel plusieurs premiers et deuxièmes éléments d'âme (40a, 40b) sont découpés à partir de la même nappe continue (40), et lesdits premiers et deuxièmes éléments d'âme (40a, 40b) sont maintenus sur des enveloppes à vide (80a, 80b), lesdites enveloppes à vide (80a, 80b) étant agencées autour de la circonférence d'un tambour de rotation (60), ledit tambour étant mis en rotation autour d'un axe principal,
dans lequel l'âme absorbante est formée en mettant en rotation chacun des premiers et deuxièmes éléments d'âme (40a, 40b) autour d'axes (A) perpendiculaires au plan de l'élément d'âme (40a, 40b), et en combinant, par la suite, un premier élément d'âme (40a) et un deuxième élément d'âme (40b) selon une relation proximale afin de former l'âme absorbante,
**caractérisé en ce qu'**une came d'élévation d'enveloppe agit de manière à élever l'enveloppe à vide (80a, 80b) radialement vers l'extérieur à partir dudit tambour (60) afin d'appliquer les éléments d'âme (40a, 40b) sur une nappe de réception (1) à une étape de transfert.

2. Procédé selon la revendication 1, dans lequel chacun des premiers et deuxièmes éléments d'âme (40a, 40b) est mis en rotation sur 90° dans des directions de rotation opposées l'une à l'autre.

3. Procédé selon les revendications 1 ou 2, dans lequel la nappe continue (40) est constituée de fibres appliquées par jets d'air ou de mousse, de préférence, de mousse.

4. Procédé selon l'une quelconque des revendications 1 à 3, dans lequel une nappe de réception (1) est acheminée par rapport à un cadre fixe (35) suivant une trajectoire amont (3), une trajectoire aval (5), et une trajectoire intermédiaire (7a, 7b, 7c) comprise entre la trajectoire amont (3) et la trajectoire aval (5), la nappe (1) ayant suivant la trajectoire amont (3) et suivant la trajectoire aval (5) une vitesse de transport sensiblement constante, dans lequel la nappe de réception (1) passe le long de rouleaux de guidage (9, 11) amont et aval qui sont fixes, du point de vue d'une translation, par rapport au cadre (35), et le long de rouleaux de transport (13, 15) amont et aval qui se déplacent de manière périodique par rapport aux rouleaux de guidage (9, 11) autour d'une position de transfert (39), et
dans lequel les éléments d'âme (40a, 40b) distincts sont appliqués sur la nappe de réception (1) lorsque celle-ci se trouve à la position de transfert.

5. Procédé selon la revendication 4, dans lequel le déplacement périodique des rouleaux de transport (13, 15) autour de la position de transfert se produit à une fréquence comprise entre 1 Hz et 100 Hz, de préférence, entre 1 Hz et 10 Hz.

6. Procédé selon l'une quelconque des revendications précédentes,
dans lequel la nappe de réception (1) comprend, soit une feuille de dessus perméable aux liquides, soit une feuille de fond imperméable aux liquides.

7. Appareil (38) pour la fabrication d'âmes absorbantes jetables à partir d'une nappe continue (40), l'appareil comprenant des moyens pour découper plusieurs premiers et deuxièmes éléments d'âme (40a, 40b) à partir de la même nappe continue (40), et l'appareil comprend, en outre, des enveloppes à vide (80a, 80b) qui sont agencées autour de la circonférence d'un tambour de rotation (60), les premiers et deuxièmes éléments d'âme (40a, 40b) étant maintenus sur lesdites enveloppes à vide (80a, 80b), chacune des enveloppes à vide (80b) pouvant être mise en rotation autour d'un axe (A) perpendiculaire au plan des éléments d'âme (40a, 40b), l'appareil comprenant, en outre, un tambour de rotation (60) afin d'appliquer des premiers et deuxièmes éléments d'âme distincts (40a, 40b) sur une nappe de réception (1), combinant, ainsi, un premier élément d'âme (40a) et un deuxième élément d'âme (40b) selon une relation proximale afin de former l'âme absorbante,
**caractérisé en ce que** l'appareil comprend, en outre, une came d'élévation d'enveloppe capable d'élever l'enveloppe à vide (80a, 80b) radialement vers l'extérieur à partir dudit tambour (60) afin d'appliquer les éléments d'âme (40a, 40b) sur la nappe de réception (1) à une étape de transfert.
